# EUROPEAN PATENT APPLICATION

(11) **EP 1 859 788 A1**
(43) Date of publication of application: **28.11.2007**
(21) Application number: 06010740.6
(22) Date of filing: 24.05.2006
(51) Int. Cl.: A61K 9/20, A61K 9/28, A61K 9/00

(54) **Production of enveloped pharmaceutical dosage forms**

(71) Applicant: Abbott GmbH & Co. KG, 65205 Wiesbaden (DE)
(72) Inventor: Rosenberg, Jörg, 67158 Ellerstadt (DE); Breitenbach, Jörg, 68199 Mannheim (DE); Hach, Harald, 76889 Oberottenbach (DE); Westedt, Ulrich, 69198 Schriesheim (DE); Knobloch, Martin, 67141 Neuhofen (DE)
(74) Representative: Reitstötter - Kinzebach

(57) **Abstract**

A process for at least partially enveloping a pharmaceutical dosage form, in which the dosage form is surrounded by a shrinkable film, and the film is subsequently shrunk is described.

## Description

The present invention relates to a process for at least partially enveloping pharmaceutical dosage forms.

Pharmaceutical forms coated with a covering layer are known from the literature. The applied covering layer has substantially one or more of the following objects:
a) taste masking: a covering layer can be used in order for example to mask the bitter taste of an active ingredient;
b) stabilization: covering layers can improve the storage stability of the dosage form, e.g. by protecting a light- or moisture-sensitive active ingredient from environmental influences;
c) identification: colored covering layers facilitate recognition and differentiation of dosage forms;
d) control: the rate of delivery of the active ingredient from the pharmaceutical form can be controlled by water-insoluble, pH-dependently soluble and/or water-permeable covering layers.

In many cases, control of delivery of active ingredient is effected almost completely by the applied covering layer, while the underlying shaped article releases the active ingredient rapidly per se. The integrity of the covering layer thus has a very special importance. If the covering layer is damaged, has insufficient thickness etc, delivery of the active ingredient may be undesirably fast. This is dangerous in the particular case of slow-release pharmaceutical forms because they generally comprise large amounts of active ingredient which are intended to be delivered over a prolonged period. If the entire amount of active ingredient is released too quickly, this may lead to considerable side effects.

In the case of dosage forms produced by extrusion, the covering layer can be applied for example by coextrusion, meaning the use of two extruders which run in parallel and with which one of the extruders provides the material of the covering layer, and the other provides that of the active ingredient-containing core. A process of this type is described in EP 0857062. However, this process is very complicated because of the strict requirements of good manufacturing practice (GMP) which must be complied with in the drugs sector. A further possibility for applying a coating layer to dosage forms produced by extrusion is the calendering process described in WO 96/19963, where the active ingredient-containing melt is introduced between two films of the enveloping material into the calender molding rolls. Since the films are sealed at the peripheral line, however, a seam is formed at this point, which is visually disadvantageous. In addition, this process cannot be used if the extrudate is only an intermediate product and is compressed to the finished dosage form only after grinding and compression with further excipients.

Another possibility for applying the covering layer to dosage forms is film coating, in which a solution of the coating material is applied to the dosage form, e.g. by spraying on, and the solvent is subsequently evaporated. However, it is disadvantageous that organic solvents have to be employed for enveloping the dosage forms with water-insoluble coatings. The process is unsuitable for porous or mechanically unstable forms. In addition, tablets can be only completely coated with the process. Orifices in the film coating layer, through which active ingredient release takes place in the case of water-insoluble coatings, are, as described in EP 0294993, added only in a second process step, thus making the process likewise very complicated.

The invention is based on the object of indicating an alternative process which permits at least partial enveloping of the surface of a pharmaceutical dosage form, preferably with mechanically stable covering layers. The process is intended simultaneously to permit, by choosing different degrees of envelopment of the dosage form, targeted adjustment of different active ingredient release profiles, for example an active ingredient release adapted to the biological rhythm.

The present invention relates to a process for at least partially enveloping a pharmaceutical dosage form, in which the dosage form is surrounded by a shrinkable film, and the film is subsequently shrunk.

The film can be applied to the dosage form in various ways. Thus, for example, a ribbon of shrinkable film can be wound spirally around the dosage form so that the ribbon preferably overlaps at the edges; the tablet body can be introduced between an upper sheet and lower sheet of shrinkable film, and the upper sheet and lower sheet can subsequently be sealed along the peripheral line; a further possibility is to form a loop in a sheet of shrinkable film, to introduce the dosage form, and to seal the film sheet behind it. In these ways, the dosage form is initially surrounded by a comparatively loose film covering. The shrinking process then attaches the film firmly to the surface of the dosage form.

However, the dosage form is preferably introduced into a tubular film or a section of a tubular film, and then this film is directly attached firmly to the surface of the dosage formed by shrinkage. The internal diameter of the tubular film is preferably chosen so that it is slightly larger than the (maximum) diameter of the dosage form. It is possible in this way for the dosage form easily to be pushed into the tubular film.

The shrinkage of the film is initiated by exposure to energy, particularly preferably exposure to thermal energy, e.g. by introducing the dosage form surrounded by the shrinkable film into a stream of hot air.

A shrinkable film or shrink film means a synthetic film which contracts on exposure to energy, in particular on heating. The films are normally made of thermoplastic synthetic materials which are subjected to a tension, less often a pressure, in one or two directions (mono- or uniaxial or biaxial stretching) in the solid state or during solidification, resulting in an increase in the dimensions by a factor of up to 10. This process is referred to as stretching. In the stretching there is an increased parallel alignment (orientation) of the chain segments of the macromolecules of amorphous regions of the polymers. If crystallization of the oriented regions is avoided, the films contract and return to their original condition on later thermal treatment. The desire of the molecules of the synthetic material to return to their original tension-free arrangement is called the recall capacity or elastic shape memory.

Suitable as material for the shrink films are all cold-stretched thermoplastic synthetic films which contract and return to their original state on input of energy. Preference is given here to polymers which can be used for pharmaceutical applications and which can be processed to films, if appropriate after admixture of suitable excipients.

The polymers which can be used may be divided into water-insoluble polymers and those which are substantially water-soluble or swellable or colloidally dispersible in water. Water-soluble, water-swellable and water-dispersible are referred to hereinafter collectively as substantially water-soluble polymers.

Suitable substantially water-soluble polymers which can be used pharmaceutically are, for example:
homopolymers and copolymers of N-vinyllactams, in particular homopolymers and copolymers of N-vinylpyrrolidone, e.g. polyvinylpyrrolidone (PVP), copolymers of N-vinylpyrrolidone and vinyl acetate or vinyl propionate,
cellulose esters and cellulose ethers, in particular methylcellulose and ethylcellulose, hydroxyalkylcelluloses, in particular hydroxypropylcellulose, hydroxyalkylalkylcelluloses, in particular hydroxypropylmethylcellulose, cellulose phthalates or succinates, in particular cellulose acetate phthalate and hydroxypropylmethylcellulose phthalate, hydroxypropylmethylcellulose succinate or hydroxypropylmethylcellulose acetate succinate;
high molecular weight polyalkylene oxides such as polyethylene oxide and polypropylene oxide and copolymers of ethylene oxide and propylene oxide,
polyacrylates and polymethacrylates such as methacrylic acid/ethyl acrylate copolymers, methacrylic acid/methyl methacrylate copolymers, butyl methacrylate/2-dimethylaminoethyl methacrylate copolymers, poly(hydroxyalkyl acrylates), poly(hydroxyalkyl methacrylates), polyacrylamides,
vinyl acetate polymers such as copolymers of vinyl acetate and crotonic acid, partially hydrolyzed polyvinyl acetate (also referred to as partially hydrolyzed "polyvinyl alcohol"),
polyvinyl alcohol.

Suitable water-insoluble polymers which can be used pharmaceutically are, for example:
polyolefins such as polyethylene, polypropylene;
polyvinyl chloride;
polyvinyl acetate.

Preference is given to the substantially water-soluble polymers of the cellulose ether and ester type, the polyacrylates and polymethacrylates. Particular preference is given according to the invention to hydroxypropylmethylcellulose, hydroxypropylcellulose and ethylcellulose.

Preferred among the water-insoluble polymers are polyethylene, polypropylene and polyvinyl chloride.

Mixtures of the abovementioned polymers are also possible, the decisive factor being a sufficiently high elasticity of the produced film so that cold stretching thereof is possible without tearing, and it is shrinkable again on subsequent heating. Any inadequate elasticity can be improved by admixture of plasticizers. Suitable plasticizers are, for example, long-chain alcohols, ethylene glycol, propylene glycol, glycerol, trimethylolpropane, triethylene glycol, butanediols, pentanols, such as pentaerythritol, hexanols, polyethylene glycols, polypropylene glycols, polyethylene/propylene glycols, silicones, aromatic carboxylic esters (e.g. dialkyl phthalates, trimellithic esters, benzoic esters, terephthalic esters) or aliphatic dicarboxylic esters (e.g. dialkyl adipates, sebacic esters, azelaic esters, citric and tartaric esters), fatty acid esters such as glycerol monoacetate, glycerol diacetate or glycerol triacetate or sodium diethyl sulfosuccinate.

The shrinking temperature of the shrinkable film is preferably between 60°C and 300°C, preferably between 70°C and 200°C and particularly preferably between 80°C and 150°C. The use of films with shrinking temperatures above 300°C is not precluded according to the invention. Such temperatures are, however, suitable only in exceptional cases because of the thermal stress on the dosage form and because of the thermal instability of many active pharmaceutical ingredients.

Both mono- and biaxially stretched films are suitable for the process. Monoaxially stretched films are preferably employed in the case of a tubular film because they permit the shrink film to be applied to the shaped article of the dosage form in a simpler and more wrinkle-free manner. A measure of the stretching of the film is its shrinkage behavior in the longitudinal and radial directions. In preferred embodiments, a film with a radial shrinkage of 30-80% and a longitudinal shrinkage of ≤ 15% is used, particularly preferably a film with radial shrinkage of 40-70% and longitudinal shrinkage of ≤ 10%.

The thickness of the films used is in the 1-100 µm range, preferably in the 5-50 µm range.

On use of substantially water-soluble film materials, altering the thickness of the shrink film allows the active ingredient release from the dosage forms produced according to the invention to be controlled.

Use of thicker films may be worthwhile to increase the mechanical stability of pharmaceutical forms, e.g. if the resistance of the pharmaceutical forms to crushing is inadequate. It may also be worthwhile for preventing pharmaceutical misuse, e.g. by mechanical manipulation of a tablet, such as grinding, and subsequent extraction of the active ingredients, to employ thick and elastic films which make grinding of the tablet difficult. One advantage of the process of the invention compared with film-coating processes is that thick layers can be applied to the dosage forms just as quickly and easily as thinner layers, whereas long process times would result in film coating.

The process of the invention allows the degree of envelopment of the dosage form to be altered easily. The degree of envelopment of the dosage form is intended to mean the proportion of the total surface of the dosage form which is enveloped surface, i.e. the ratio of enveloped surface and total surface. It is possible by altering the degree of envelopment to control the rate and/or time course of active ingredient release. In preferred embodiments, at least 25% of the surface of the pharmaceutical dosage form is covered with the film, and in other preferred embodiments at least 50% or at least 75%.

A further possibility is to increase the surface of the dosage form available for active ingredient release by subsequent treatment of the enveloped dosage form, e.g. by a pinned roller.

The release characteristics can additionally be controlled by altering the geometric shape of the dosage form. In a preferred embodiment, the dosage form has a substantially cylindrical shape and the lateral surface of the cylinder is covered with a water-insoluble film, leaving both end faces exposed. It is possible in this way to achieve virtually zero water release when the active ingredient is delivered from the dosage form. Since the active ingredient release can in these cases take place only via the end faces of the cylindrical dosage forms, the rate of active ingredient delivery depends, for the same dose, ultimately only on the ratio of the area of these cylinder end faces to the length of the forms. It is thus possible by altering the length and the diameter to generate virtually any desired active ingredient releases. The ratio between length and diameter of the dosage forms enveloped according to the invention is preferably in the range from 0.5 : 1 to 8 : 1, preferably 0.8 : 1 to 6 : 1, particularly preferably 1 : 1 to 3 : 1.

Dosage forms with biphasic active ingredient release can be achieved by reducing the degree of envelopment of the cylindrical dosage forms by, for example, not enveloping parts of the lateral surface, besides the end faces. The unenveloped part of the dosage form shows initially fast release of the active ingredient (burst effect), while delivery of the active ingredient from the enveloped part is uniform and slow. The proportions of active ingredient released quickly and slowly can be adapted virtually as desired by altering the proportion of the unenveloped surface compared with the enveloped surface. Such biphasic active ingredient releases are conceivable for example for headache therapy: fast initial dosage for acute therapy, followed by a maintenance dose to maintain the freedom from pain.

The underlying dosage form which is at least partly enveloped by the process of the invention can be obtained in various ways. For example, it can be a tablet body obtained by tableting, e.g. a compressed powder or granules. The dosage forms to be enveloped can be produced in a conventional way by compressing powders or granules. The processes described in the literature for producing solid pharmaceutical forms can be employed for this purpose, in particular the technologies described for producing tablets.

The tablet body is preferably obtained by a melt process, e.g. a melt extrudate. Production starts from a mixture which comprises one or more active pharmaceutical ingredients and one or more excipients and which, through melting or softening of at least one component, becomes a paste or viscous liquid and therefore extrudable.

These are in particular mixtures which comprise pharmacologically acceptable polymers, for example
homopolymers and copolymers of N-vinyllactams, in particular homopolymers and copolymers of N-vinylpyrrolidone, e.g. polyvinylpyrrolidone (PVP), copolymers of N-vinylpyrrolidone and vinyl acetate or vinyl propionate,
cellulose esters and cellulose ethers, in particular methylcellulose and ethylcellulose, hydroxyalkylcelluloses, in particular hydroxypropylcellulose, hydroxyalkylalkylcelluloses, in particular hydroxypropylmethylcellulose, cellulose phthalates or succinates, in particular cellulose acetate phthalate and hydroxypropylmethylcellulose phthalate, hydroxypropylmethylcellulose succinate or hydroxypropylmethylcellulose acetate succinate;
high molecular weight polyalkylene oxides such as polyethylene oxide and polypropylene oxide and copolymers of ethylene oxide and propylene oxide,
polyacrylates and polymethacrylates such as methacrylic acid/ethyl acrylate copolymers, methacrylic acid/methyl methacrylate copolymers, butyl methacrylate/2-dimethylaminoethyl methacrylate copolymers, poly(hydroxyalkyl acrylates), poly(hydroxyalkyl methacrylates),
polyacrylamides,
vinyl acetate polymers such as copolymers of vinyl acetate and crotonic acid, partially hydrolyzed polyvinyl acetate (also referred to as partially hydrolyzed polyvinyl alcohol),
polyvinyl alcohol,
oligo- and polysaccharides such as carrageenans, galactomannans and xanthans, or mixtures of one or more thereof.

Of these, homo- or copolymers of vinylpyrrolidone are particularly preferred, e.g. polyvinylpyrrolidone with Fikentscher K values of from 12 to 100, preferably 17 to 30, or copolymers of from 30 to 70% by weight N-vinylpyrrolidone (VP) and 70 to 30% by weight vinyl acetate (VA), such as, for example, a copolymer of 60% by weight VP and 40% by weight VA.

It is, of course, also possible to employ mixtures of said polymers.

Active ingredients mean in the context of the invention all substances with a desired physiological effect on the human or animal body or plants. They are in particular active pharmaceutical ingredients. The amount of active ingredient per dose unit can vary within wide limits. It is ordinarily chosen to be sufficient to achieve the desired effect. It is also possible to employ combinations of active ingredients. Active ingredients in the context of the invention are also vitamins and minerals.

Examples of suitable active substances include, but are not limited to:
analgesic and anti-inflammatory drugs such as fentanyl, indomethacin, ibuprofen, naproxene, diclofenac, diclofenac sodium, fenoprofen, acetylsalicylic acid, ketoprofen, nabumetone, paracetamol, piroxicam, meloxicam, tramadol, and COX-2 inhibitors such as celecoxib and rofecoxib;
anti-arrhythmic drugs such as procainamide, quinidine and verapamil;
antibacterial and antiprotozoal agents such as amoxicillin, ampicillin, benzathine penicillin, benzylpenicillin, cefaclor, cefadroxil, cefprozil, cefuroxime axetil, cephalexin, chloramphenicol, chloroquine, ciprofloxacin, clarithromycin, clavulanic acid, clindamycin, doxyxycline, erythromycin, flucloxacillin sodium, halofantrine, isoniazid, kanamycin sulphate, lincomycin, mefloquine, minocycline, nafcillin sodium, nalidixic acid, neomycin, nortloxacin, ofloxacin, oxacillin, phenoxymethyl-penicillin potassium, pyrimethamine-sulfadoxime and streptomycin;
anti-coagulants such as warfarin;
antidepressants such as amitriptyline, amoxapine, butriptyline, clomipramine, desipramine, dothiepin, doxepin, fluoxetine, reboxetine, amineptine, selegiline, gepirone, imipramine, lithium carbonate, mianserin, milnacipran, nortriptyline, paroxetine, sertraline and 3-[2-[3,4-dihydrobenzofuro[3,2-c]pyridin-2(1 H)-yl]ethyl]-2-methyl-4H-pyrido[1,2-a]pyrimidin-4-one;
anti-diabetic drugs such as glibenclamide and metformin;
anti-epileptic drugs such as carbamazepine, clonazepam, ethosuximide, gabapentin, lamotrigine, levetiracetam, phenobarbitone, phenytoin, primidone, tiagabine, topiramate, valpromide and vigabatrin;
antifungal agents such as amphotericin, clotrimazole, econazole, fluconazole, flucytosine, griseofulvin, itraconazole, ketoconazole, miconazole nitrate, nystatin, terbinafine and voriconazole;
antihistamines such as astemizole, cinnarizine, cyproheptadine, decarboethoxyloratadine, fexofenadine, flunarizine, levocabastine, loratadine, norastemizole, oxatomide, promethazine and terfenadine;
anti-hypertensive drugs such as captopril, enalapril, ketanserin, lisinopril, minoxidil, prazosin, ramipril, reserpine, terazosin and telmisartan;
anti-muscarinic agents such as atropine sulphate and hyoscine;
antineoplastic agents and antimetabolites such as platinum compounds, such as cisplatin and carboplatin; taxanes such as paclitaxel and docetaxel; tecans such as camptothecin, irinotecan and topotecan; vinca alkaloids such as vinblastine, vindecine, vincristine and vinorelbine; nucleoside derivatives and folic acid antagonists such as 5-fluorouracil, capecitabine, gemcitabine, mercaptopurine, thioguanine, cladribine and methotrexate; alkylating agents such as the nitrogen mustards, e.g. cyclophosphamide, chlorambucil, chiormethine, iphosphamide, melphalan, or the nitrosoureas, e.g. carmustine, lomustine, or other alkylating agents, e.g. busulphan, dacarbazine, procarbazine, thiotepa; antibiotics such as daunorubicin, doxorubicin, idarubicin, epirubicin, bleomycin, dactinomycin and mitomycin; HER 2 antibodies such as trastuzumab; podophyllotoxin derivatives such as etoposide and teniposide; famesyl transferase inhibitors; anthrachinon derivatives such as mitoxantron;
anti-migraine drugs such as alniditan, naratriptan and sumatriptan;
anti-Parkinsonian drugs such as bromocryptine mesylate, levodopa and selegiline;
antipsychotic, hypnotic and sedating agents such as alprazolam, buspirone, chlordiazepoxide, chlorpromazine, clozapine, diazepam, flupenthixol, fluphenazine, flurazepam, 9-hydroxyrisperidone, lorazepam, mazapertine, olanzapine, oxazepam, pimozide, pipamperone, piracetam, promazine, risperidone, selfotel, seroquel, sertindole, sulpiride, temazepam, thiothixene, triazolam, trifluperidol, ziprasidone and zolpidem;
anti-stroke agents such as lubeluzole, lubeluzole oxide, riluzole, aptiganel, eliprodil and remacemide;
antitussives such as dextromethorphan and laevodropropizine;
antivirals such as acyclovir, ganciclovir, loviride, tivirapine, zidovudine, lamivudine, zidovudine/lamivudine, didanosine, zalcitabine, stavudine, abacavir, lopinavir, amprenavir, nevirapine, efavirenz, delavirdine, indinavir, nelfinavir, ritonavir, saquinavir, adefovir and hydroxyurea;
beta-adrenoceptor blocking agents such as atenolol, carvedilol, metoprolol, nebivolol and propanolol;
cardiac inotropic agents such as amrinone, digitoxin, digoxin and milrinone;
corticosteroids such as beclomethasone dipropionate, betamethasone, budesonide, dexamethasone, hydrocortisone, methylprednisolone, prednisolone, prednisone and triamcinolone;
disinfectants such as chlorhexidine;
diuretics such as acetazolamide, furosemide, hydrochlorothiazide and isosorbide;
enzymes;
essential oils such as anethole, anise oil, caraway, cardamom, cassia oil, cineole, cinnamon oil, clove oil, coriander oil, dementholised mint oil, dill oil, eucalyptus oil, eugenol, ginger, lemon oil, mustard oil, neroli oil, nutmeg oil, orange oil, peppermint, sage, spearmint, terpineol and thyme;
gastro-intestinal agents such as cimetidine, cisapride, clebopride, diphenoxylate, domperidone, famotidine, lansoprazole, loperamide, loperamide oxide, mesalazine, metoclopramide, mosapride, nizatidine, norcisapride, olsalazine, omeprazole, pantoprazole, perprazole, prucalopride, rabeprazole, ranitidine, ridogrel and sulphasalazine;
haemostatics such as aminocaproic acid;
lipid regulating agents such as atorvastatin, fenofibrate, fenofibric acid, lovastatin, pravastatin, probucol and simvastatin;
local anaesthetics such as benzocaine and lignocaine;
opioid analgesics such as buprenorphine, codeine, dextromoramide, dihydrocodeine, hydrocodone, oxycodone and morphine;
parasympathomimetics and anti-dementia drugs such as AIT-082, eptastigmine, galanthamine, metrifonate, milameline, neostigmine, physostigmine, tacrine, donepezil, rivastigmine, sabcomeline, talsaclidine, xanomeline, memantine and lazabemide;
peptides and proteins such as antibodies, becaplermin, cyclosporine, tacrolimus, erythropoietin, immunoglobulins and insuline;
sex hormones such as oestrogens: conjugated oestrogens, ethinyloestradiol, mestranol, oestradiol, oestriol, oestrone; progestogens; chlormadinone acetate, cyproterone acetate, 17-deacetyl norgestimate, desogestrel, dienogest, dydrogesterone, ethynodiol diacetate, gestodene, 3-keto desogestrel, levonorgestrel, lynestrenol, medroxy-progesterone acetate, megestrol, norethindrone, norethindrone acetate, norethisterone, norethisterone acetate, norethynodrel, norgestimate, norgestrel, norgestrienone, progesterone and quingestanol acetate;
stimulating agents such as sildenafil, vardenafil;
vasodilators such as amlodipine, buflomedil, amyl nitrite, diltiazem, dipyridamole, glyceryl trinitrate, isosorbide dinitrate, lidoflazine, molsidomine, nicardipine, nifedipine, oxpentifylline and pentaerythritol tetranitrate;
their N-oxides, their pharmaceutically acceptable acid or base addition salts and their stereochemically isomeric forms.

Pharmaceutically acceptable acid addition salts comprise the acid addition salt forms which can be obtained conveniently by treating the base form of the active ingredient with appropriate organic and anorganic acids.

Active ingredients containing an acidic proton may be converted into their non-toxic metal or amine addition salt forms by treatment with appropriate organic and inorganic bases.

The term addition salt also comprises the hydrates and solvent addition forms which the active ingredients are able to form. Examples of such forms are hydrates, alcoholates and the like.

The N-oxide forms of the active ingredients comprise those active ingredients in which one or several nitrogen atoms are oxidized to the so-called N-oxide.

The term "stereochemically isomeric forms" defines all possible stereoisomeric forms which the active ingredients may possess. In particular, stereogenic centers may have the R- or S-configuration and active ingredients containing one or more double bonds may have the E- or Z-configuration.

The composition may additionally comprise various optional excipients. Such optional excipients are:
plasticizers such as, for example, C₇-C₃₀-alkanols, ethylene glycol, propylene glycol, glycerol, trimethylolpropane, triethylene glycol, butanediols, pentanols such as pentaerythritol and hexanols, polyalkylene glycols, preferably having a molecular weight of from 200 to 1000, such as, for example, polyethylene glycols, polypropylene glycols and polyethylene/propylene glycols, silicones, aromatic carboxylic esters (e.g. dialkyl phthalates, trimellithic esters, benzoic esters, terephthalic esters) or aliphatic dicarboxylic esters (e.g. dialkyl adipates, sebacic esters, azelaic esters, citric and tartaric esters), fatty acid esters such as glycerol monoacetate, glycerol diacetate or glycerol triacetate or sodium diethyl sulfosuccinate. The concentration of plasticizer where present is generally from 0.5 to 30, preferably 0.5 to 10, % by weight based on the total weight of polymer and plasticizer. The amount of plasticizer is advantageously not more than 30% by weight based on the total weight of polymer and plasticizer so that - in the area of solid forms - there is formation of storage-stable formulations and dosage forms which show no cold flow.

Sugar alcohols such as sorbitol, xylitol, mannitol, maltitol; or sugar alcohol derivatives such as isomalt or hydrogenated condensed palatinose as described in DE 102 62 005.

Solubilizers such as sorbitan fatty acid esters, polyalkoxylated fatty acid esters such as, for example, polyalkoxylated glycerides, polyalkoxylated sorbitan fatty acid esters or fatty acid esters of polyalkylene glycols, or polyalkoxylated ethers of fatty alcohols. A fatty acid chain in these compounds ordinarily comprises from 8 to 22 carbon atoms. The polyalkylene oxide blocks comprise on average from 4 to 50 alkylene oxide units, preferably ethylene oxide units, per molecule.

Suitable sorbitan fatty acid esters are sorbitan monolaurate, sorbitan monopalmitate, sorbitan monostearate, sorbitan monooleate, sorbitan tristearate, sorbitan trioleate, sorbitan monostearate, sorbitan monolaurate or sorbitan monooleate.

Examples of suitable polyalkoxylated sorbitan fatty acid esters are polyoxyethylene (20) sorbitan monolaurate, polyoxyethylene (20) sorbitan monopalmitate, polyoxyethylene (20) sorbitan monostearate, polyoxyethylene (20) sorbitan monooleate, polyoxyethylene (20) sorbitan tristearate, polyoxyethylene (20) sorbitan trioleate, polyoxyethylene (4) sorbitan monostearate, polyoxyethylene (4) sorbitan monolaurate or polyoxyethylene (4) sorbitan monooleate.

Suitable polyalkoxylated glycerides are obtained for example by alkoxylation of natural or hydrogenated glycerides or by transesterification of natural or hydrogenated glycerides with polyalkylene glycols. Commercially available examples are polyoxyethylene glycerol ricinoleate 35, polyoxyethylene glycerol trihydroxystearate 40 (Cremophor® RH40, BASF AG) and polyalkoxylated glycerides like those obtainable under the proprietary names Gelucire® and Labrafil® from Gattefosse, e.g. Gelucire® 44/14 (lauroyl macrogol 32 glycerides prepared by transesterification of hydrogenated palm kernel oil with PEG 1500), Gelucire® 50/13 (stearoyl macrogol 32 glycerides, prepared by transesterification of hydrogenated palm oil with PEG 1500) or Labrafil M1944 CS (oleoyl macrogol 6 glycerides prepared by transesterification of apricot kernel oil with PEG 300).

A suitable fatty acid ester of polyalkylene glycols is, for example, PEG 660 hydroxystearic acid (polyglycol ester of 12-hydroxystearic acid (70 mol%) with 30 mol% ethylene glycol).

Suitable polyalkoxylated ethers of fatty alcohols are, for example, macrogol 6 cetylstearyl ether or macrogol 25 cetylstearyl ether

Solubilizers are typically included in the powder mixture in an amount of from 0.1 to 15% by weight, preferably 0.5 to 10% by weight.

Disintegrants such as crosslinked polyvinylpyrrolidone and crosslinked sodium carboxymethylcellulose.

Extenders or fillers such as lactose, cellulose, silicates or silica,

Lubricants such as magnesium stearate and calcium stearate, sodium stearylfumarate,

Colorants such as azo dyes, organic or inorganic pigments or colorants of natural origin,

Stabilizers such as antioxidants, photostabilizers, hydroperoxide destroyers, free-radical scavengers, stabilizers against microbial attack.

It is expedient to mix the components or some of the components of the melt before the heating to give a power mixture. The mixing of the components to give the powder mixture takes place in conventional mixers such as plowshare mixers, agitating or free-fall mixers and the like.

The heating of the powder mixture takes place in an apparatus usual for this purpose. Particularly suitable are heatable extruders or kneaders such as mixer/kneader reactors (e.g. ORP, CRP, AP, DTB from List or Reactotherm supplied by Krauss-Maffei or Ko-kneader supplied by Buss), trough mixers and internal mixers or rotor/stator systems (e.g. Dispax supplied by IKA). The residence time of the composition in the extruder is preferably less than 5 minutes, in particular less than 3 minutes.

Extruders which can be employed are single-screw machines, intermeshing screw machines or else multiscrew extruders, in particular twin-screw extruders. The screws may be corotating or counter-rotating and equipped if appropriate with mixing and/or kneading elements such as kneading disks. Corotating twin-screw extruders are particularly preferred.

The charging of the extruder or kneader takes place continuously or batchwise according to the design thereof in a conventional way. The powder mixture is preferably fed in freely, e.g. through a weigh feeder. It is also possible to meter in one or more of the starting materials in liquid form, i.e. dissolved or dispersed, for example through liquid pumps within the extrusion section.

The use of continuously operating kneaders or extruders is preferred. This entails the powdered mixture of the polymer and of the active ingredient being introduced at one terminal entry into an elongate extruder housing; the mixture being heated in order to obtain a melt; the melt being conveyed through the extruder housing to a terminal exit of the extruder housing; and a sufficient back pressure being generated in the extruder housing for the melt to emerge as a coherent extrudate at one terminal exit from the extruder housing.

The resulting composition is then subjected according to the invention to a shaping. It is possible in this connection to produce a large number of shapes depending on the tool and mode of shaping. It is preferred in the case of the process of the invention to discharge the active ingredient-containing melt from round-section dies so that extrudates of defined thickness are obtained and become set on a downstream conveyor belt, where appropriate with cooling. Single cylindrical pieces can be cut out of these extrudates while still in the plastic state, but also after setting, and are optimally suitable for applying a covering layer by the process of the invention by shrinking of a piece of shrink tubing.

In some circumstances, these cold melt forms can also be ground to powder and then compressed to tablets in a conventional way. It is possible to include in this case tableting aids such as colloidal silicon dioxide, calcium hydrogen phosphate, lactose, microcrystalline cellulose, starch or magnesium stearate.

The tablets obtained thereby or else the forms obtained by extrusion with subsequent shaping step are subsequently introduced entirely or with only part of their surface into a tubular shrink film. The shrink film is then directly shrunk onto the dosage form firmly and wrinkle-free by thermal or radiation energy.

The invention is illustrated in more detail by the following examples and the appended drawings.

Fig. 1 shows the active ingredient release from the enveloped extrudate pieces as a function of the release time.

Fig. 2a and fig. 2b show the active ingredient release from enveloped paracetamol tablets in 0.01 molar hydrochloric acid solution and 20% strength aqueous ethanol solution, respectively.

Fig. 3a to fig. 3c show enveloped paracetamol tablets with different degrees of envelopment.

### Examples:

### Example 1: Production of extrudates

A powder mixture consisting of 65.0% clarithromycin and 20.2% by weight hydroxypropylcellulose (type: Klucel EF) was processed at room temperature with a mixture consisting of 7.0% by weight propylene glycol monolaurate (Lauroglycol FCC, F. Gattefossee) and 7.8% by weight in a mixer (supplied by Diosna) to homogeneous granules. These granules were metered through a weigh feeder into a twin-screw extruder. Extrusion took place at a temperature of 130°C, and it was possible to discharge a white homogeneous extrudate from the round-section die (diameter 5 mm) of the extruder. After a short cooling time in air, the extrudate had a diameter of about 6 mm and could easily be cut manually while still in the plastic state into cylindrical pieces.

### Example 2: Enveloping the extrudate pieces

The extrudate pieces obtained as in example 1 were put into tubular polyolefin shrink films of equal length (technical specification: shrinking temperature >125°C, radial shrinkage: 50%, longitudinal shrinkage: max. 10%), whose internal diameter was in each case about 1 mm larger than the diameter of the extrudate pieces. This was followed by treatment with a hot-air blower for a few seconds, thus shrinking the film completely and smoothly onto the outer surface of the extrudates. The upper and lower end face of the cylindrical extrudates remained free.

Enveloped extrudate pieces with a length of 4.5 mm, 9.0 mm, 18.0 mm and 36.0 mm were produced (the diameter of the unenveloped extrudate was 6 mm in all cases).

### Example 3: Testing of active ingredient release from the enveloped extrudate pieces

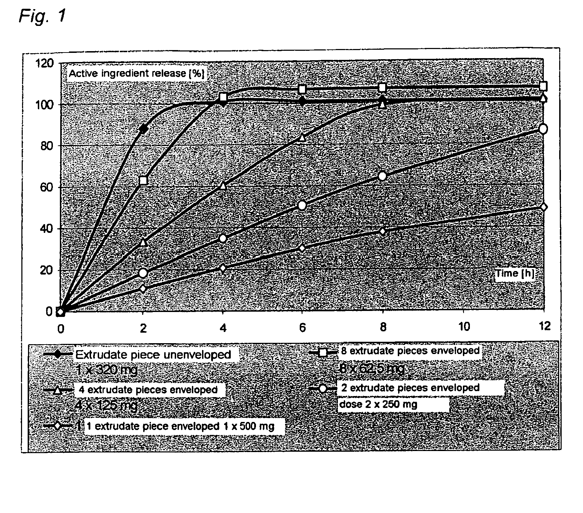

The active ingredient release from the enveloped extrudate pieces from part B was tested with the aid of the method according to the American pharmacopeia (USP 28; apparatus 2) at 37°C, with the stirrer revolving at 50 rpm and in 900 ml of a potassium dihydrogenphosphate buffer solution (0.05 mol/l, adjusted to pH 5.5). The active ingredient release was in each case tested with the same amount of active ingredient, i.e. the number of enveloped extrudates present in the test vessel was in each case such that a total dose of 500 mg of active ingredient resulted. The amount of active ingredient delivered into the buffer solution from the extrudate pieces at each time of testing was determined by HPLC. The results are summarized in table 1 below and in fig. 1.

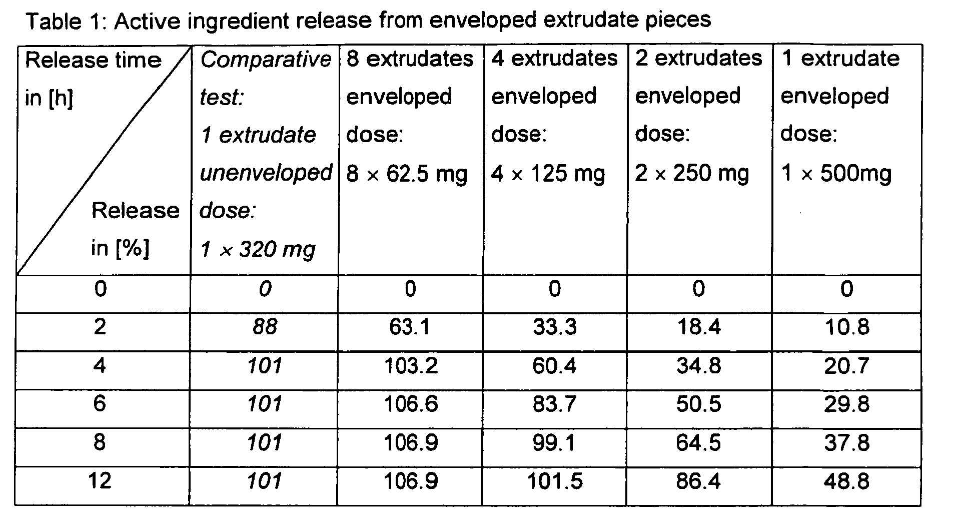

### Example 4: Production of paracetamol tablets

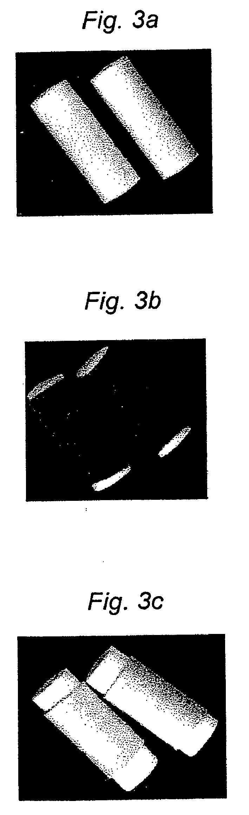

Directly compressible paracetamol granules ("DC-90 material") consisting of 90.0% by weight paracetamol, 6.5% by weight starch (Starch 1500), 2% by weight polyvinylpyrrolidone (Kollidon^{®} 25, BASF), 1% by weight crospovidone (Kollidon^{®} CL, BASF) and 0.5% by weight stearic acid were compressed in a tablet press to elongate cylindrical tablets (diameter 6 mm, length 18 mm). The tablets obtained in this way are shown in fig. 3a.

### Example 5: Enveloping the paracetamol tablets with shrink film

The tablets obtained in example 5 were completely enveloped on their cylindrical surface with a polyolefin shrink film (diameter about 7 mm). The shrink film was shrunk on by briefly heating the films with a hot-air blower, after which the film was firmly attached to the cylindrical tablet. Only the upper and lower end face of the cylindrical tablets remained free. The active ingredient dosage in the tablets was 500 mg of paracetamol, and the total weight including the covering layer was 560 mg. The tablets obtained in this way are shown in fig. 3b.

### Example 6: Partial enveloping of the paracetamol tablets with shrink film

The enveloping with the shrink film took place as indicated in example 5, but in this case only 12 mm of the length of the cylindrical tablets was enveloped with the shrink film, the remaining tablet body remained free. The tablets obtained in this way are shown in fig. 3b.

### Example 7: Testing of the active ingredient release from the enveloped paracetamol tablets

The active ingredient release from the enveloped paracetamol tablets from examples 4 to 6 was tested with the aid of the method according to the American pharmacopeia (USP 28; apparatus 2) at 37°C, with the stirrer rotating at 50 rpm and in 900 ml of a 0.01 molar hydrochloric acid solution and a 20% strength aqueous ethanol solution. The amount of active ingredient delivered into the buffer solution from the extrudate pieces at each time of testing was determined by HPLC.

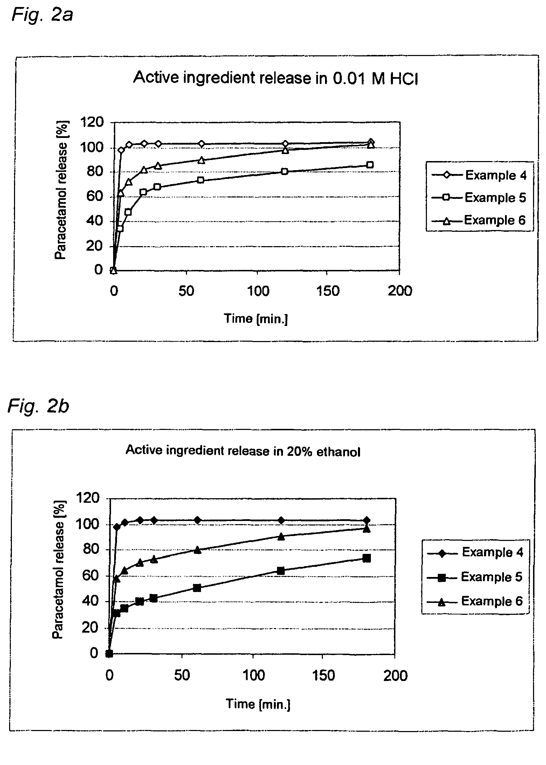

The measured active ingredient releases are summarized in table 2 below and figures 2a and 2b.

**Table2 : Active ingredient releases from the pharmaceutical forms of example 4 to 6**

| Time [min] | **Active ingredient release in 0.01 M HCl [%]** | | | **Active ingredient release in 20% ethanol [%]** | | |
|---|---|---|---|---|---|---|
| | **Example 4** | **Example 5** | **Example 6** | **Example 4** | **Example 5** | **Example 6** |
| 0 | **0** | **0** | **0** | **0** | **0** | **0** |
| 5 | **98** | **34** | **63** | **98** | **31** | **58** |
| 10 | **102** | **47** | **72** | **101** | **35** | **64** |
| 20 | **103** | **63** | **82** | **103** | **40** | **70** |
| 30 | **103** | **68** | **85** | **103** | **43** | **73** |
| 60 | **103** | **73** | **90** | **103** | **51** | **80** |
| 120 | **103** | **80** | **98** | **103** | **64** | **91** |
| 180 | **104** | **85** | **102** | **103** | **74** | **97** |

It is clear from the results of the active ingredient release that the unenveloped tablets deliver the contained active ingredient very rapidly to the aqueous medium. By contrast, the enveloping with the shrink film leads to a marked retardation of the rate of delivery of the active ingredient. This slower release of the active ingredient also remains stable in 20% ethanol, i.e. in a medium in which the paracetamol is distinctly more soluble than in 0.01 M HCl.

A biphasic delivery of the active ingredient is possible in particular by partial enveloping of the cylindrical tablet surface if a part of this surface is not enveloped by the shrink film (example 6).

## Claims

1. A process for at least partially enveloping a pharmaceutical dosage form, in which the dosage form is surrounded by a shrinkable film, and the film is subsequently shrunk.

2. The process as claimed in claim 1, in which at least 25% of the surface of the pharmaceutical dosage form is covered with the film.

3. The process as claimed in claim 1 or 2, in which the pharmaceutical dosage form is introduced into a tubular shrinkable film.

4. The process as claimed in any of claims 1 to 3, in which a film with a shrinking temperature of 60-300°C is used.

5. The process as claimed in claim 4, in which a film with a shrinking temperature of 70-200°C is used.

6. The process as claimed in claim 5, in which a film with a shrinking temperature of 80-150°C is used.

7. The process as claimed in any of claims 3 to 6, in which a film with a radial shrinkage of 30-80% and a longitudinal shrinkage of 5 15% is used as film.

8. The process as claimed in claim 7, in which a film with a radial shrinkage of 40-70% and a longitudinal shrinkage of ≤ 10% is used as film.

9. The process as claimed in any of the preceding claims, where the film comprises at least one water-insoluble polymer.

10. The process as claimed in claim 9, where the water-insoluble polymer is selected from the group of polyethylene, polypropylene, polyvinyl chloride and mixtures thereof.

11. The process as claimed in any of claims 1 to 8, where the film comprises at least one substantially water-soluble polymer.

12. The process as claimed in claim 11, in which the substantial water-soluble polymer is selected from the group of hydroxypropylcellulose, hydroxypropylmethylcellulose, ethylcellulose and mixtures thereof.

13. The process as claimed in any of the preceding claims, in which the shrinking of the film is achieved by exposure to heat or radiation.

14. The process as claimed in any of the preceding claims, where the pharmaceutical dosage form comprises at least one active ingredient and at least one binder.

15. The process as claimed in claim 14, where the pharmaceutical dosage form is a tablet body obtained by tableting.

16. The process as claimed in claim 14, where the pharmaceutical dosage form is a tablet body obtained by a melt process.

17. The process as claimed in any of claims 14 to 16, where the pharmaceutical dosage formed has a substantially cylindrical shape, and the lateral surface of the cylinder is covered with a water-insoluble film.

18. A pharmaceutical dosage form obtained by a process as claimed in any of claims 14 to 17, having a substantially constant and pH-independent release of active ingredient.
